# EUROPÄISCHE PATENTSCHRIFT

(11) **EP 2 613 758 B1**
(45) Veröffentlichungstag und Bekanntmachung des Hinweises auf die Patenterteilung: **16.11.2016**
(21) Anmeldenummer: 11758159.5
(22) Anmeldetag: 02.09.2011
(51) Int. Cl.: A61K 8/06, A61K 8/49, A61Q 15/00

(54) **MAKROEMULSIONEN MIT VERBESSERTER DEODORANTWIRKSAMKEIT**
MACROEMULSIONS WITH IMPROVED DEODORANT EFFICACY
MACRO-ÉMULSIONS AYANT UN EFFET DÉODORANT AMÉLIORÉ

(30) Priorität: 09.09.2010 DE 102010044787
(43) Veröffentlichungstag der Anmeldung: 17.07.2013
(73) Patentinhaber: Beiersdorf AG, 20253 Hamburg (DE)
(72) Erfinder: MEYER, Maren, 22459 Hamburg (DE); BIEL, Stefan, 21077 Hamburg (DE); WEINERT, Katrin, 22763 Hamburg (DE); TSCHIERSKE, Nicole, 20146 Hamburg (DE)
(74) Vertreter: Hartmann, Jost
(86) Internationale Anmeldenummer: PCT/EP2011/065190
(87) Internationale Veröffentlichungsnummer: WO 2012/031984

(56) Entgegenhaltungen:
- WO-A1-2007/141289
- WO-A1-2009/148947
- WO-A2-2009/106468
- DE-A1-102004 049 282
- DE-A1-102004 051 420
- DE-A1-102008 001 811
- DE-C1- 4 240 674
- US-A1- 2008 221 165
- US-A1- 2008 254 084
- N.-O. HÜBNER, J. SIEBERT, A. KRAMER: "Octenidine Dihydrochloride, a Modern Antiseptic for Skin, Mucous Membranesand Wounds", SKIN PHARMACOLOGY AND PHYSIOLOGY, Bd. 23, 28. Mai 2010 (2010-05-28), Seiten 244-258, XP002671250, Basel DOI: 10.1159/000314699 in der Anmeldung erwähnt

## Beschreibung

Die Erfindung umfasst kosmetische oder dermatologische Zubereitungen auf Basis einer Makroemulsion umfassend ein oder mehrere amphiphile Deodorantwirkstoffe, dadurch gekennzeichnet, dass als amphiphiler Deodorantwirkstoff Octenidindihydrochlorid gewählt wird.

Kosmetische oder dermatologische Zubereitungen, insbesondere in Form von Roll-on, Zerstäuber und Aerosolen, als Makroemulsion formuliert umfassend ein oder mehrere amphiphile Deodorantwirkstoffe zeigen eine verbesserte desodorierende Wirkleistung als vergleichbar formulierte Mikroemulsionen.
Emulsionen sind thermodynamisch instabile Dispersionen zweier nicht miteinander mischbarer Flüssigkeiten. Die innere (diskontinuierliche) Phase ist in Form von Tröpfchen in der äußeren (kontinuierlichen) Phase verteilt. Ist die innere Phase die lipophile Phase, so handelt es sich um eine Öl-in-Wasser-Emulsion (O/W-Emulsion). Bei einer Wasser-in-ÖI-Emulsion (W/O-Emulsion) ist die lipophile Phase die äußere Phase. Es sind auch Mehrfachemulsionen vom Typ W/O/W und O/W/O bekannt.
Ein weiteres Unterscheidungsmerkmal für Emulsionen ist die Einteilung in Mikro- und Makroemulsionen. In vielen Dokumenten werden auch Nanoemulsionen beschrieben, die wiederum eine Untergruppe der Mikroemulsionen darstellen. Unterscheidungskriterium der Mikro- und Makroemulsion ist die Tropfengröße. So erscheinen Makroemulsionen weiß, da die Tropfengröße größer ist als die Wellenlänge des sichtbaren Lichts. Mit Verringerung der Tropfendurchmesser werden die Emulsionen immer transluzenter, bis hin zu klaren mizellaren Lösungen.
Die "Makroemulsion" ist eine weiße Emulsion, die beispielsweise mit einem Steareth-2/Steareth-21-Emulgatorsystem, welches flüssigkristalline Strukturen ausbildet, oder mit Polyglyceryl-3 Methylglucose Distearat hergestellt werden kann. Die Emulsion ist selbstverdickend.
Aufgrund ihrer Festkörpereigenschaften zeigen Flüssigkristalle Auffälligkeiten in verschiedenen Untersuchungsmethoden. Sie sind doppelbrechend und daher im polarisierten Licht sichtbar. Mit Hilfe von Streu- und Beugungsmethoden, z.B. Röntgenkleinwinkelstreuung, kann die Aggregationsform ermittelt werden. Die magnetische Anisotropie der Flüssigkristalle führt im NMR-Spektrum zur Verbreiterung von Signalen und zu Grundlinienverkrümmungen. Rheologische Parameter geben Aufschluss über die Festigkeit der Strukturen und damit über die Stärke intermolekularer Wechselwirkungen.

Zum Unterschied sind Mikroemulsionen thermodynamisch stabile Emulsionen mit einer großen Öl/Wasser-Grenzfläche. Aus diesem Grund benötigen sie hohe Mengen an Emulgatoren. Das Resultat sind transluzente bis transparente Emulsionen mit Tropfengrößen von 10 bis 50 nm. Sie unterliegen einer sehr hohen Dynamik in Bezug auf ihre innere Struktur.
Die "Mikroemulsion" ist eine transluzente, beispielsweise durch ein PIT-Verfahren (englisch: phase inversion temperature) hergestellte Emulsion, welche beispielsweise mit einem Assoziativverdicker (PEG-150 Distearate) verdickt werden kann.

Zur Unterscheidung der beiden Emulsionsarten eignet sich die Bestimmung der Tropfengrößen. Für Tropfengrößen im Bereich 0,02 bis 2000 µm ist die Laserbeugung eine geeignete Methode. In diesem Bereich der Monomer-Tröpfchen liegen die Makroemulsionen.
Für Emulsionen mit Tropfengrößen von 1 bis 1000 nm wird die Photonenkorrelationsspektroskopie verwendet, den Bereich, in denen die Mikroemulsionen liegen.
So gelten Emulsionen mit einer Monomer-Tröpfchengröße von 1 bis 10 µm als Makroemulsionen und Emulsionen mit einem Tröpfchendurchmesser von 0,005 bis 0,1 µm als Mikroemulsion (Römpp-Chemie Lexikon, März 2002). Diese Einteilung wird auch erfindungsgemäß genutzt.
Eine Emulsion mit einer Tröpfchengrößenverteilung im Übergangsbereich zwischen 0,1 bis 1 µm wird erfindungsgemäß je nach den weiteren Unterscheidungskriterien entweder als Mikro- oder Makroemulsion definiert.
Grundsätzlich dienen neben der Tröpfchengröße die Kriterien Opazität und Bildung flüssigkristalliner Strukturen zur Einteilung als Mikro- oder Makroemulsion.
Mikroemulsionen sind transluzente bis transparente Emulsionen. Makroemulsionen weisen flüssigkristalline Phasen auf, während die Mikroemulsion keine Überstrukturen zeigt.

Kosmetische Antitranspirantien oder Desodorantien/Deodorantien dienen dazu, Körpergeruch zu beseitigen bzw. deren Entstehung zu vermindern. Körpergeruch durch Schweiß entsteht, wenn der an sich geruchlose frische Schweiß durch Mikroorganismen wie z.B. Staphylokokken und Corynebakterien zersetzt wird.
Den üblichen kosmetischen Desodorantien liegen unterschiedliche Wirkprinzipien zugrunde. Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruchs (Geruchsverbesserungsmittel). Gängige Wirkmechanismen hierzu sind antibakterielle Effekte, wie sie auch das nicht-kolloidale Silber zeigt, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Reaktionen zu wohlriechenden Stoffen umgesetzt werden. Zubereitungen können neben den eigentlichen schweißhemmenden Wirkstoffen (AT-Wirker) zusätzlich auch Stoffe enthalten, die den mikrobiellen Abbau des Schweißes hemmen, wie z.B. Triclosan. Triclosan wirkt gegen gram-positive und gram-negative Keime sowie gegen Pilze und Hefen, woraus eine desodorierende, jedoch keine antitranspirante Wirkung resultiert, da aus der Beeinflussung der bakteriellen Hautflora keine Beeinflussung der Schweißsekretion abzuleiten ist.
Schweißgeruch besteht zu einem Großteil aus verzweigtkettigen Fettsäuren, die durch bakterielle Enzyme aus geruchlosem Schweiß freigesetzt werden. Klassische Deo-Wirkstoffe wirken dem entgegen, indem sie das Wachstum von Bakterien reduzieren. Häufig wirken die dabei zum Einsatz kommenden Substanzen jedoch unselektiv auch gegen nützliche Hautkeime und können bei empfindlichen Personen zu Hautirritationen führen.

Octenidindihydrochlorid, CAS 70775-75-6 (N, N'- (1,10-decanediyldi-1[4H]-pyridinyl-4-ylidene)-bis-(octanamine) dihydrochloride, Octenidine HCl, Octenidine) der Struktur 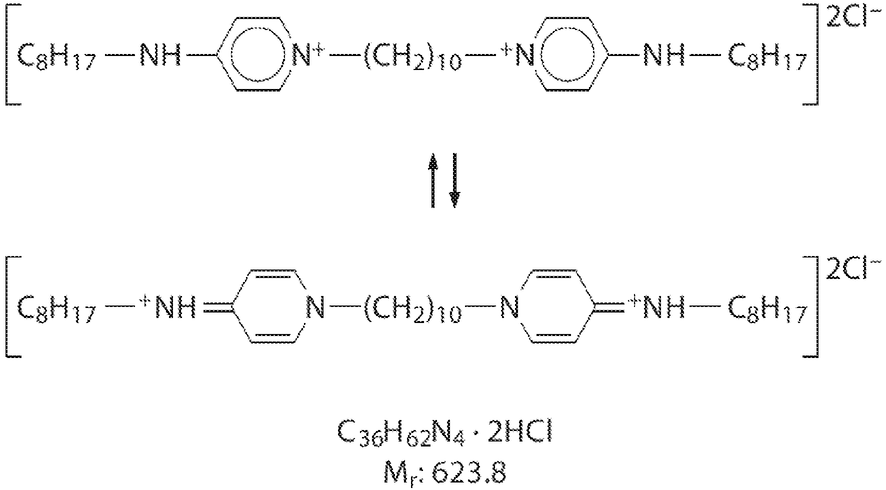 ist als oberflächenaktive, amphiphile, antimikrobiell wirksame Substanz bekannt (Skin Pharmacol Physiol 2010; 23, 244 - 258).
Eine Anti-Biofilm-Wirksamkeit von Octenidine HCl ist ebenfalls vorbeschrieben.

Bekannt sind Mischungen aus Ethylhexylglycerin, Octenidine HCl und Propylenglykol unter der Handelsbezeichnung Sensidin DO.

Aus der DE 424 06 74 C1 ist bekannt, dass Glycerinmonoalkylether der Formel ROCH₂CHOHCH₂OH eine gute desodorierende Wirkung besitzen. Vorzugsweise wird 1-(2-Ethylhexyl)glycerinether eingesetzt (Handelsprodukt Sensiva® SC 50, Schülke & Mayr GmbH). Aus dieser Patentschrift ist u.a. eine Deoformulierung bekannt, die 58 Gew.-% Wasser, 40 Gew.-% Ethanol und 1,0 Gew.-% 1,2-Propylenglykol, 0,9 Gew.-% 2-Ethylhexylglycerinether und 0,1 Gew.-% Bispyridiniumalkan (z.B. Octenidindihydrochlorid) umfasst. Diese Formulierung zeigt sich als nicht hinreichend lagerstabil. Es kann dabei zur Bildung von Peroxiden und in deren Folge zur Bildung von Abbauprodukten kommen, wie flüchtigen Substanzen mit geringem Molekulargewicht und unangenehm riechenden Alkoholen, wie 2-Ethylhexanol. Zudem sind der Gehalt an flüchtigen Substanzen (VOC, wie Ethanol) und der Wirkstoffgehalt in der fertigen Zubereitung vergleichsweise hoch.

DE 10 2008 011692 A1 offenbart klare, farblose, wasser- bzw. alkoholhaltige Lösungen mit Bispyridiniumalkanen, wie Octenidindihydrochlorid, die in Deodorantzubereitungen eingesetzt werden können. Als eine bevorzugte Ausführungsform der Zubereitungen für die topische Anwendung sind Emulsionen (z. B. Cremes, Lotionen), Gele, Lösungen,

Einreibemittel, Salben, Sprays oder Puder genannt. Bevorzugt sind dennoch klare wasser-oder alkoholhaltige Reinigungszubereitungen.
Der konkrete Einsatz von Octenidindihydrochlorid in Makroemulsionen wird nicht offenbart.

DE 10 2005 002644 A1 offenbart Zusammensetzungen für die hygienische Händedesinfektion oder Händewaschung, wobei die Zusammensetzung Glycerinmonomethylether, ein oder mehrere Bispyridiniumalkane sowie gegebenenfalls ein oder mehrere Polyole und/oder ein oder mehrere Tenside umfasst.
Die Wasserlöslichkeit von Glycerinmonomethylether wird hierin durch Zusatz des Bispyridiniumalkans, insbesondere Octenidindihydrochlorid, signifikant verbessert.
Der Einsatz von Octenidindihydrochlorid in Makroemulsionen wird nicht offenbart.

WO 2008/ 052 912 A1 offenbart antimikrobielle Mundwässer mit Octenidindihydrochlorid. Die Zubereitungen sind wässrige Lösungen, die typischerweise eine Phase bilden und klar sind.

US-A-2008/0254084 beschreibt eine Creme, die eine Makroemulsion darstellt, enthaltend 0,05 Gew.% Octenidinhydrochlorid.

US-A-2008/0221165 beschreibt Makroemulsionen, z.B. als Creme oder ointment, enthaltend 0,1 bis 0,3 Gew.% Octenidinhydrochlorid.

WO 2009/106468 A1 zeigt, dass 1-(2- Ethylhexyl)-glycerolether als desodorierender Wirkstoff bekannt ist und Mischungen aus Octenidin und Ethylhexylglycerin als Desinfektionsmittel eingesetzt werden können. Weiterhin wird gezeigt, dass wässrige Lösungen bevorzugte Zubereitungsformen sind und der Anteil an Octenidin über 0,2 Gew.% liegen sollte.

Die meisten antibakteriellen Substanzen, die in Desodorantien zur Verminderung der geruchsverursachenden Bakterien eingesetzt werden, sind aus wässrigen oder wässrigalkoholischen Formulierungen heraus gut wirksam. In einigen Emulsionen hingegen treten viele Deo-Wirkstoffe aufgrund ihrer amphiphilen Struktur mit den Emulgatoren in Wechselwirkung. Dabei werden sie in das Netzwerk zwischen Öl- und Wasserphase eingebunden und stehen für die antimikrobielle Wirksamkeit nicht mehr zur Verfügung. Eine desodorierende Wirkung kann daher in vielen emulsionsbasierten Zubereitungen nicht mehr festgestellt werden.

Amphiphil bedeutet, dass ein Molekül sowohl einen polaren (hydrophilen) als auch einen unpolaren (lipophilen) Teil enthält. Diese Eigenschaft führt dazu, dass sich Amphiphile an Grenzflächen (Wasser/Luft, Wasser/Öl) anreichern. Die betreffenden Grenzflächen werden durch diese monomolekularen Adsorptionsschichten in ihren physikochemischen Eigenschaften verändert, z.B. durch Erniedrigung der Grenzflächenspannung.

Octenidindihydrochlorid ist eine oberflächenaktive, amphiphile Verbindung.

Wünschenswert ist es, kosmetische oder dermatologische desodorierende Zubereitungen auf Emulsionsbasis zur Verfügung zu stellen, in denen auch amphiphile Deowirkstoffe eine ausreichende Freisetzung zeigen.

Die Erfindung umfasst kosmetische oder dermatologische Zubereitungen, entsprechend Anspruch 1, auf Basis einer Makroemulsion, d.h. beispielsweise mit einer Tropfengröße im Bereich von etwa 1 bis 10 µm, umfassend ein oder mehrere amphiphile Wirkstoffe, insbesondere desodorierende Wirkstoffe, dadurch gekennzeichnet, dass als amphiphiler Wirkstoff Octenidindihydrochlorid gewählt wird.

Die die Erfindung betreffende Makroemulsion weist als besonders vorteilhaftes Merkmal ein flüssigkristallines System auf, deren Flüssigkristalle, auch Mesophasen genannt, Eigenschaften flüssiger und fester Zustände besitzen. Sie sind fließfähig (Flüssigkeitseigenschaft), wobei ihre Moleküle positions- oder orientierungsgeordnet vorliegen und dadurch optische Anisotropie zu beobachten ist (Kristalleigenschaft). Wird der Phasenübergang in ein flüssigkristallines System durch Temperaturänderung induziert, handelt es sich um thermotrope Flüssigkristalle. Ist die Zugabe von Lösungsmitteln die Ursache, werden sie als lyotrope Flüssigkristalle bezeichnet. Lyotrope Flüssigkristalle resultieren aus der Selbstorganisation von amphiphilen Verbindungen. Amphiphile Moleküle sind im Fall der Makroemulsion beispielsweise die nichtionischen Emulgatoren Steareth-2 und Steareth-21.
Vorteilhaft basieren die kosmetischen oder dermatologischen Zubereitungen daher auf einer Makroemulsion, wobei der Tropfengrößendurchmesser der Emulsionstropfen im Bereich von 1 bis 10 µm liegt und die Emulsion ein flüssigkristallines System ausbilden kann.
Die erfindungsgemäße Emulsion bildet auch ohne Octenidine ein flüssigkristallines System aus. Dieses System wird durch Zugabe von Octenidine nicht zerstört, so dass durch Einarbeitung von Octenidine in eine Makroemulsion eine solche mit flüssigkristallinen System erhalten bzw. erzeugt wird.
In Untersuchungen von Gloor et al. (2002) konnte gezeigt werden, dass sich die *in vivo-*Wirkung eines antimikrobiellen Wirkstoffs, welcher sich in der Ölphase einer Formulierung löst, erheblich von der Wirkung eines in der wässrigen Phase gelösten Wirkstoffs unterscheidet. Nach Gloor et al. (2002) befinden sich die Bakterien in einem wässrigen Milieu auf der Haut. Beim Auftragen einer Formulierung auf die Haut vermischt sich zunächst die Wasserphase der Formulierung mit dem wässrigen Milieu der Haut und in der Wasserphase gelöste Wirkstoffe können unmittelbar auf die Bakterien wirken. Lipophile Wirkstoffe, die sich in der Ölphase einer Formulierung befinden, müssen erst in den wässrigen Bereich diffundieren und zeigen entsprechend eine schlechtere bzw. verzögerte Wirksamkeit. Das bedeutet, dass die Wirksamkeit eines Produkts auf Emulsionsbasis unter anderem davon abhängig ist, wo sich der Wirkstoff in der Emulsion befindet.
Um eine gute Wirksamkeit zu besitzen, sollte sich ein Wirkstoff demnach in ausreichender Konzentration in der Wasserphase befinden. Die ist u.a. auch der Grund, warum viele desodorierende Zubereitungen als wässrige bzw. wässrig-alkoholische Lösungen vorliegen. In Form einer Wasser-in-Öl Emulsion ist ein hydrophiler Wirkstoff in der Wasserphase gelöst und muss nach der Auftragung auf die Haut erst durch den umgebenden Ölfilm hindurch, um auf der Haut Wirkung zeigen zu können. Dies führt zu einem Wirkverlust bei W/O-Emulsionen.

Demnach sollte sich bei einer Emulsion der Wirkstoff ebenfalls in ausreichender Konzentration in der Wasserphase einer Emulsion befinden, um eine gute Wirksamkeit zu besitzen.

Die Lokalisation von Wirkstoffen in einer Formulierung kann demgemäß Vorhersagen über die Wirksamkeit des Produkts liefern.

In Untersuchungen hat sich gezeigt, dass grenzflächenaktive, amphiphile Wirkstoffe wie Benzethonium chloride und Octenidine HCl/Ethylhexylglycerin in einer Mikroemulsion unwirksam sind.
Es ist bekannt, dass lipophile Wirkstoffe in der lipophilen Zwischenschicht gebunden sind bzw. die Ölphase einer Emulsion als Wirkstoffreservoir fungiert. Wasserlösliche Wirkstoffe hingegen diffundieren durch die hydrophilen Bereiche zwischen den Emulgatornetzwerkstrukturen, während amphiphile Wirkstoffe zwischen den Strukturen und durch diese hindurch diffundieren können (Kreuter, 1994). Damit ist der direkte Weg des Wirkstoffs innerhalb der Emulsion aber durch das Emulgatornetzwerk blockiert.
Jeder Wirkstoff ist in einer Emulsion in allen Bereichen zu finden, je nach Struktur allerdings zu unterschiedlichen Anteilen: Lipophile Wirkstoffe halten sich bevorzugt in der Ölphase, hydrophile in der Wasserphase auf. Amphiphile Wirkstoffe, wie Octenidine HCl, können sich in Öl/Wasser-Grenzflächen oder Emulgatornetzwerken einlagern bzw. selbst Mizellen bilden.

Die mögliche Ursache für die Unwirksamkeit oder zumindest Schwächung der Wirkung amphiphiler Wirkstoffe aus Mikroemulsionen könnte darin liegen, dass die Tropfen in Mikroemulsionen thermodynamisch stabil sind und sie auch beim Auftragen auf die Haut nicht zerstört werden. Damit kann eine Freisetzung von in Tropfen bzw. in der Grenzfläche befindlichen amphipilen Wirkstoffen nur über Diffusion stattfinden. Da auf der Haut der Wassergehalt sehr niedrig ist, besteht nach kurzer Zeit kein treibender Konzentrationsgradient zwischen wässrigem Milieu und Tropfen mehr. Deshalb und wegen der festen Einbindung in die Grenzfläche stehen die amphipilen Wirkstoffe nicht mehr zur Reaktion mit Bakterien zur Verfügung; die Mikroemulsion mit diesen Wirkstoffen ist deshalb unwirksam. Die Lokalisation der Wirkstoffe an der Öl/Wasser-Grenzfläche wurde experimentell bestimmt, was die Unwirksamkeit der Mikroemulsion mit diesen Wirkstoffen begründet.

Den Wirkverlust kann man versuchen, durch erhöhte Zugabe an Wirkstoff, wie Octenidine, auszugleichen. Dies gelingt auch in der Mikroemulsion und es kommt bei genügend hohen Wirkstoffanteilen auch dort zur Freisetzung. Ein erhöhter Wirkstoffgehalt ist jedoch häufig nicht wünschenswert, beispielsweise aus Verträglichkeitsgründen oder Überdosierungen.

Amphiphile Wirkstoffe, wie Octenidine, verteilen sich entsprechend ihres spezifischen Verteilungskoeffizienten (abhängig von Wirkstoff, Öl und Emulgatoren) zwischen Wasserphase und Öl-Wasser-Grenzfläche. Damit liegt immer ein Teil in der Wasserphase vor (aktive Konzentration). Wird nun der Gesamtgehalt an Wirkstoff erhöht, erhöht sich auch automatische die aktive Konzentration. Somit ist oder wird die Emulsion trotz teilweiser Einbindung des Wirkstoffs in die Grenzfläche wirksam.

Bei der vorteilhaften Anteilsmenge von 0,005 - 0,015 Gew.-% an amphiphilen Wirkstoff, insbesondere Octenidine, ist die aktive Wirkkonzentration in Mikroemulsionen aber zu niedrig. Der Teil des Wirkstoffs, der sich in der Grenzfläche befindet, erreicht die Bakterien nicht, weil die Tropfen (trotz nichtionischer Emulgatoren) eine negative Gesamtladung tragen und daraus repulsive Wechselwirkungen mit der ebenfalls negativ geladenen Bakterienzellwand resultieren.
Als bevorzugter Anteilsbereich an amphiphilen Wirkstoffen, insbesondere Octenidine, in Mikroemulsionen sind daher mindestens 0,03 Gew.-%, bezogen auf die Gesamtmasse der Zubereitung, zu wählen.

Überraschenderweise zeigen diese amphiphilen Wirkstoffe nun jedoch aus einer Makroemulsion eine ausreichende Wirksamkeit. Untersuchungen der grenzflächenaktiven Wirkstoffe Benzethoniumchlorid und insbesondere Octenidine zeigen eine Wirksamkeit aus Makroemulsionen.

Es wurden dazu die Freisetzungsgeschwindigkeit aus Makro- und Mikroemulsion untersucht.

In der Makroemulsion mit flüssigkristallinem System wurden die amphiphilen Wirkstoffe ebenfalls im Emulgatornetzwerk, welches eine ausgedehnte Grenzfläche darstellt, lokalisiert. Dass diese Formulierungen auf Makroemulsionsbasis trotzdem gut antimikrobiell wirksam sind, steht zunächst im Widerspruch zu den Untersuchungen zu Mikroemulsionen und den angeführten Begründungen.

Es zeigte sich überraschenderweise, dass die amphiphilen Wirkstoffe in Grenzflächen dann wirksam sein können, wenn die Grenzflächen beim Auftragen auf die Haut zerstört werden können und der Wirkstoff so freigesetzt wird.

Die Zerstörung der Grenzfläche wurde bei Makroemulsionen im Gegensatz zu den Mikroemulsionen beobachtet. Der drastische Eingriff des Wirkstoffs in den Aufbau des Emulgatornetzwerks der Makroemulsion wurde durch rheologische Parameter sowie DSC-und SAXS-Messungen gezeigt.

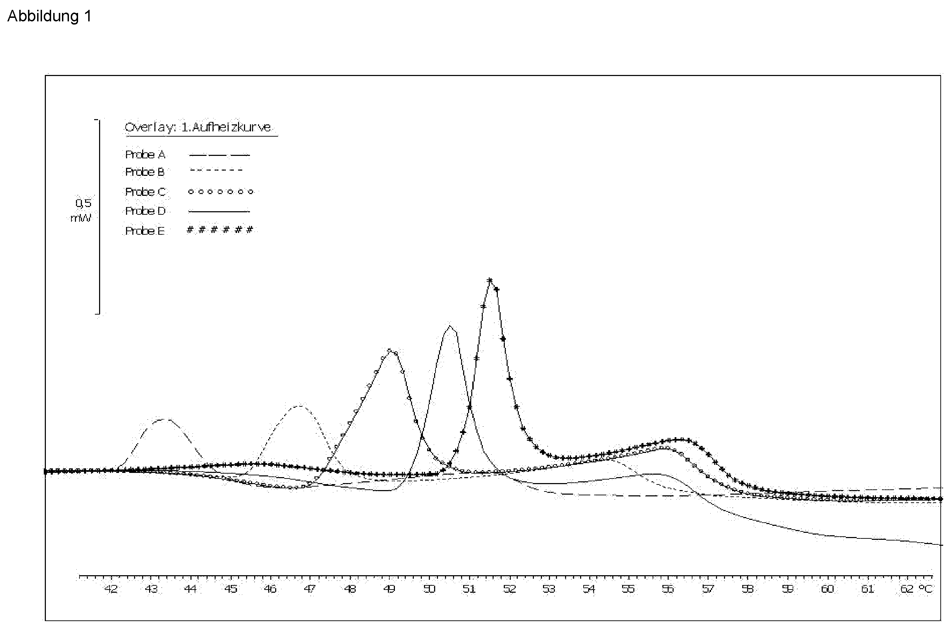

DSC-Messungen verdeutlichen den Energiebedarf zur Phasenumwandlung bei aufsteigender Temperatur. In der Abbildung 1 sind diese DSC-Messungen dargestellt. Die Proben A - D stellen Makroemulsion mit absteigender Konzentration an Octenidine HCl/ Ethylhexylglycerin dar; A - 1 %, B - 0,5%, C - 0,3%, D - 0,1 %, Probe E ist ein Makroemulsion Placebo.
Abbildung 1 zeigt, je höher die Konzentration an Wirkstoff in der Makroemulsion (Probe A, grob gestrichelter Graph), desto früher erfolgt die Phasenumwandlung, d.h. das Aufschmelzen der Flüssigkristalle wird bereits bei geringeren Temperaturen induziert. Die Lage der Peaks ist nach links verschoben.
Je höher die Konzentration an Wirkstoff in der Makroemulsion, desto weniger Energie ist für die Phasenumwandlung nötig, d.h. die Peaks weisen eine geringere Höhe auf (Probe A, grob gestrichelter Graph).
Diese frühere und energetisch unaufwändigere Phasenumwandlung der Flüssigkristalle aufgrund des höheren Anteils des Wirkstoffs zeigt eine Schwächung der Struktur im Vergleich zum Placebo, d.h. dass durch Zugabe des Wirkstoff-Gemischs die stark ausgeprägte Struktur (flüssigkristallines System) geschwächt wird und somit die Freigabe/ Freisetzung der antimikrobiellen Wirksamkeit erleichtert wird.

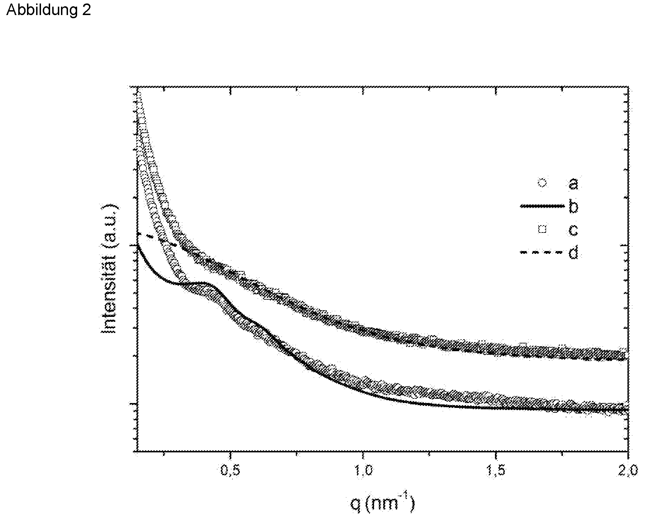

Abbildung 2 zeigt die SAXS-Messungen, Röntgenkleinwinkelstreuung, wobei a/b ein Makroemulsion Placebo und c/d eine Makroemulsion mit 0,5% Octenidine HCl/ Ethylhexylglycerin zeigt.

Es zeigt sich anhand der SAXS-Messung, dass die Makroemulsion eine ausgeprägte Überstruktur, ein flüssigkristallines System, bildet.
Durch Zugabe des Wirkstoff-Gemischs (Octenidine HCl/ Ethylhexylglycerin) verändert sich die Struktur. Ein flüssigkristallines System ist dennoch zu detektieren.
Die flüssigkristalline Struktur geht durch die Zugabe an Octenidine nicht verloren, sie wird nur geschwächt. Durch diese Schwächung wird erfindungsgemäß erst die Freisetzung des Wirkstoffs ermöglicht. Es handelt sich sozusagen um ein System, welches durch die Zugabe des Wirkstoffs eine Veränderung erfährt und so erst dessen Freigabe ermöglicht - im Gegensatz zur Mikroemulsion, bei der die Wirkstoffeinbindung in ein stabiles System so fest ist, dass keine Freisetzung mehr möglich ist.

Durch den Einsatz amphiphiler Wirkstoffe wird durch Einbindung des Wirkstoffs in das Emulgatornetzwerk dieses Netzwerk geschwächt. Deshalb wird der Wirkstoff aus der Makroemulsion im Gegensatz zur Mikroemulsion besser freigesetzt. Bei der Mikroemulsion hingegen bewirkt die Einbindung in die Öl/Wasser-Grenzfläche die Ausbildung kleinerer Tropfen. Und je kleiner ein Tropfen, desto stabiler ist dieser. Aus diesem Grund ist die Freisetzung des Wirkstoffs aus der Mikroemulsion erschwert.

Die amphiphilen Wirkstoffe, die in den Grenzflächen der Emulsionen lokalisiert wurden, sind daher nur aus Makroemulsionen wirksam. Hier gilt, dass die Wirkstoffe aus der Emulsion freigegeben werden können, wenn deren Struktur durch Scherung beim Auftragen zerstört wird.
In der bevorzugten Darreichungsform eines Aerosols wird durch die Scherung im Sprühkopf die Emulsionsstruktur beeinflusst, so dass eine verbesserte Freisetzung des Wirkstoffs, insbesondere Octenidine, beobachtet wird.

In der untersuchten Mikroemulsion waren diese Wirkstoffe unwirksam, da sie aus der stabilen Grenzfläche nicht freigesetzt werden.

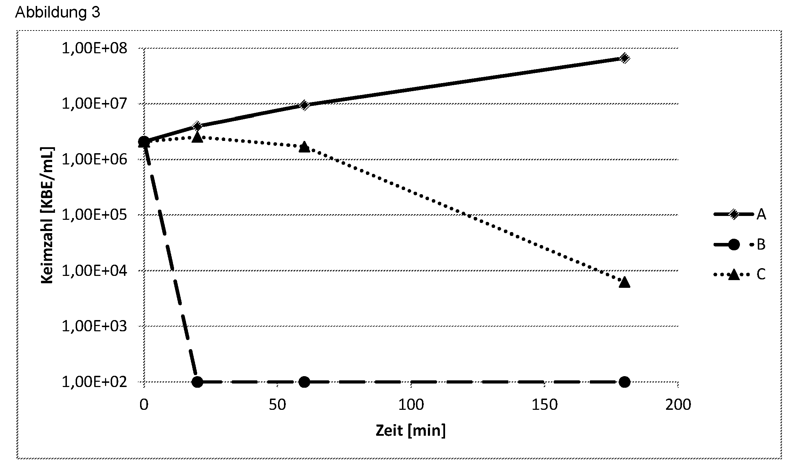

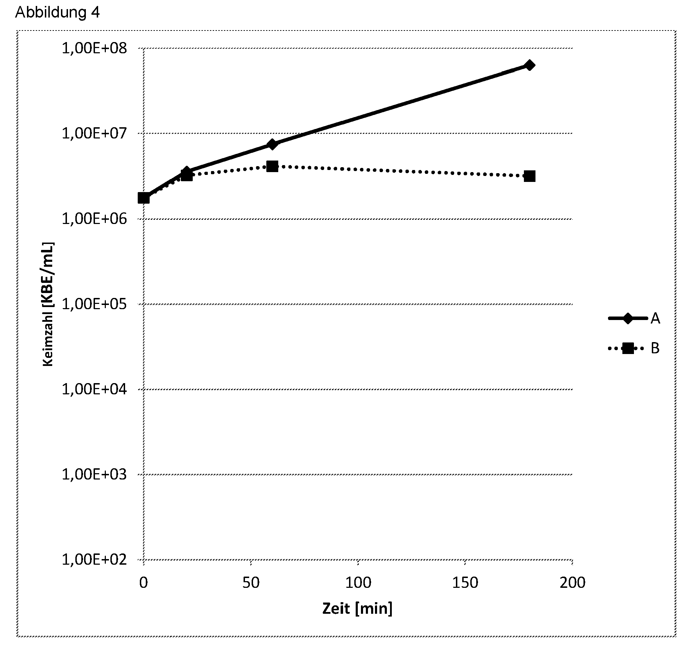

Die Wirksamkeit wurde mittels der Freisetzung von Octenidine aus Makro- bzw. Mikroemulsionen untersucht, wie es beispielhaft in Abbildung 3 und 4 dargestellt ist. Ebenso sind Untersuchungen durchgeführt worden für W/O- Aerosol-Zubereitungen einmal mit und einmal ohne Octenidine, die zu gleichen Ergebnissen geführt haben.
In den dargestellten Suspensionstests wurden zu 500µL einer Bakteriensuspension (S. *epidermidis)* 250 mg der zu testenden Formulierung gegeben (bei der Positivkontrolle nur 250 mg des Bakterienmediums). Diese Mischungen werden mit Medium auf 1000µL aufgefüllt. Die Suspension enthält dann ca. 1*10⁶ KBE/mL (KBE: koloniebildende Einheiten). Die Ansätze werden bei 37°C unter Rotation für drei Stunden inkubiert. Nach 0, 20, 60 und 180 min werden Proben genommen, verdünnt und auf Agarplatten ausplattiert. Diese werden 24h bei 37°C zur Anzucht der verbleibenden Keime inkubiert. Gute und *in vivo* wirksame Deodorant-Formulierungen reduzieren die Keimzahl bereits nach 20 Minuten auf Nachweisgrenze von 100 KBE/mL. Formulierungen mit schlechterer Keimzahl-Reduktion zeigen *in vivo* keine ausreichende Deoleistung.

Die Ergebnisse zeigen, dass die Wirkstoffe aus der Makro-Emulsion freigesetzt werden. Bei gleicher Wirkstoffkonzentration konnte bei der Mikro-Emulsion hingegen keine Wirksamkeit festgestellt werden.
Die Ergebnisse zeigen zwar, dass bei steigendem Wirkstoffgehalt letztlich auch aus der Mikro-Emulsion eine relevante Wirkleistung erzielt werden kann. Ein erhöhter Wirkstoffgehalt wirkt sich jedoch negativ auf Kosten und u.U. Verträglichkeit aus.

Die Untersuchungen zeigen, dass das Placebo alleine nicht wirksam ist (s. Abbildung 3). Ebenso ist Ethylhexylglycerin alleine in höherer Konzentration als in der angegeben Mischung nicht wirksam (s. Abbildung 4), sondern erst die Kombination mit Octenidine HCl zeigt die erfindungsgemäße Wirksamkeit (s. Abbildung 3).
Octenidine HCl alleine zeigt vorteilhaft die gewünschte Wirksamkeit.
Bevorzugt kann der Zubereitung ein Anteil von 0,05 - 0,1 Gew.-% Ethylhexylglycerin, bezogen auf die Gesamtmasse, zugesetzt werden.

Abbildung 3 zeigt die Ergebnisse des Suspensionstests. A ist ein Makroemulsions-Placebo, B eine Makroemulsion mit 0,5% Octenidine/Ethylhexylglycerin und C eine Mikroemulsion mit 0,5% Octenidine HCl/Ethylhexylglycerin.

Abbildung 4 zeigt die Ergebnisse des Suspensionstests. A ist Kontrolle und B eine Makroemulsion mit 0,5% Ethylhexylglycerin ohne Octenidine.

Zur weiteren Stütze der unterschiedlichen desodorierenden Wirkungsweise aus Mikro- bzw. Makroemulsionen wurden In-use-Tests durchgeführt. 60 Probanden, in diesem Fall weiblich, Alter 18-65 Jahre, wendeten die zu testenden Produkte täglich für sieben Tage an. Danach wird für weitere sieben Tage das jeweils andere Produkt getestet (semimonadisch). Zu jedem der zwei Produkte wird ein Fragebogen ausgefüllt, der die Zustimmung auf einer 7er Skala abbildet (1 - trifft überhaupt nicht zu, 7 - trifft voll und ganz zu).

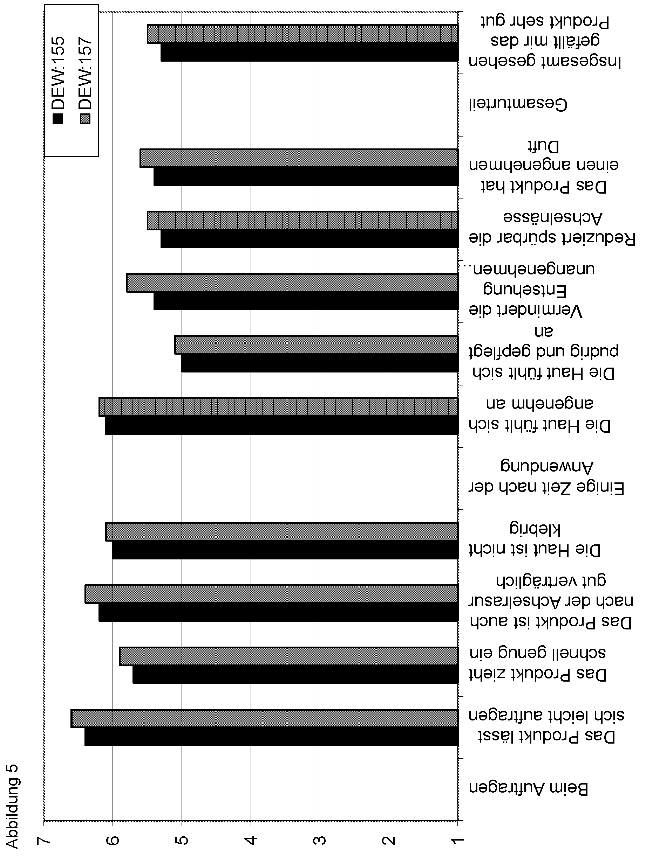

Abbildung 5 zeigt den In-use Test von Makroemulsionen. DEW 155 ist das Vergleichsprodukt, eine Makroemulsion mit Polyaminopropyl Biguanide als Deowirker. Polyaminopropyl Biguanide ist ein anerkannt guter antimikrobieller Wirkstoff.
DEW 157 ist das erfindungsgemäße Produkt, eine Makroemulsion mit Octenidine.

Der Probandentest zeigt, dass die erfindungsgemäße Zubereitung (Makro-Emulsion mit Octenidine HCl) besser abschnitt als die Vergleichszubereitung mit Polyaminopropyl Biguanide. Signifikante Unterschiede wurden für die Parameter Auftrag des Produkts sowie Entstehung des Achselgeruchs berechnet.

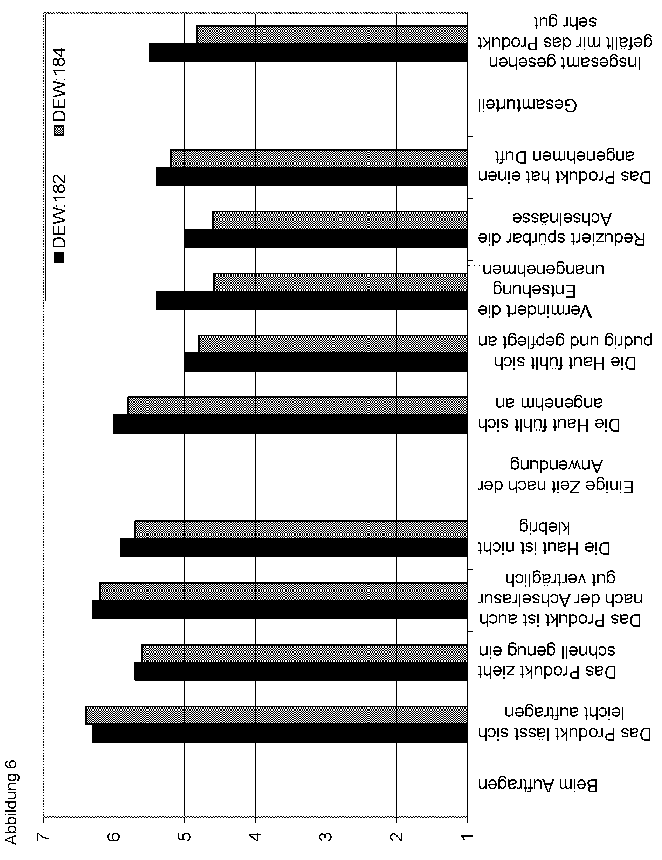

In gleicher Weise wurde anschließend die Deowirkung von Mikroemulsionen untersucht, wie Abbildung 6 zeigt.
DEW 182 ist eine Mikroemulsion mit Polyaminopropyl Biguanide als antimikrobiellem Wirkstoff, DEW 184 eine Mikroemulsion mit Octenidine.

Es zeigte sich, dass die Zubereitung mit Octenidine als Mikroemulsion dabei signifikant schlechter abschnitt als die Vergleichszubereitung mit Polyaminopropyl Biguanide.
Die beiden Untersuchungen im Vergleich wiederum zeigen eindrucksvoll, dass die Wahl der Emulsionsart, Makro- anstatt Mikroemulsion, einen wesentlichen Einfluss auf die desodorierende Wirkung ausübt.
Die in den Vergleichsuntersuchungen verwendeten Makro- bzw. Mikroemulsionen sind für den Fachmann bekannte Zubereitungen, ebenso wie die Beispiele 1 bis 5.

Die untersuchten Wirkstoffe, insbesondere Octenidine und Benzethoniumchloride, wurden des Weiteren indirekt über die Bestimmung von Viskosität und Fließgrenze, DSC-Messungen und Tropfengrößenbestimmung, sowie direkt mit NMR-Spektroskopie in den Emulsionen lokalisiert.

Die Makroemulsionen ergaben eine Viskosität von 1160 mPa*s. Durch Zugabe von 0,5 Gew.% Octenidine HCl/ Ethylhexylglycerin verringerte sich die Viskosität auf 771 mPa*s. Die geringere Viskosität bedeutet, wie zuvor dargelegt, eine Schwächung der Struktur ohne vollständigen Verlust.
Die Fließgrenze der Makroemulsion beträgt Tₘₐₓ 4,9 Pa, ηₘₐₓ 191,1 Pa*s. Durch Zugabe von 0,5 Gew.% Octenidine HCl/ Ethylhexylglycerin ergab sich Tₘₐₓ 2,1 Pa, ηₘₐₓ 36,9 Pa*s. Diese Erniedrigung der maximalen Schubspannung und der maximalen Viskosität deutet ebenfalls auf eine Schwächung der Struktur hin.

Die Mikroemulsionen zeigen Viskositäten von 372 mPa*s. Durch Zugabe von 0,5 Gew.% Octenidine HCl/ Ethylhexylglycerin ergab sich eine Zunahme der Viskosität auf 568 mPa*s. D.h. die höhere Viskosität bedeutet eine stabilere Struktur.
Die Tröpfchengrößenbestimmung der Mikroemulsion ergab 34,5 ± 2,5 nm (d₅₀). Durch Zugabe von 0,5 Gew.% Octenidine HCl/ Ethylhexylglycerin ergaben sich Tropfengrößen von 17,1 ± 1,9 nm (d₅₀), d.h. die Tröpfchen wurden kleiner. Kleinere Tropfen sind stabiler und erschweren die Wirkstoff-Freisetzung.
Demgemäß umfassen die erfindungsgemäßen kosmetischen oder dermatologischen Zubereitungen auf Basis einer Makroemulsion, d.h. insbesondere mit einer Tropfengröße im Bereich von 0,1 bis 10 µm und insbesondere mit flüssigkristalliner Struktur, ein oder mehrere amphiphile Wirkstoffe, insbesondere desodorierende Wirkstoffe, wobei als amphiphiler Wirkstoff Octenidindihydrochlorid-zu einem Anteil von 0,0001 bis 0,03 Gew.%, besonders bevorzugt im Bereich von 0,005 - 0,015 Gew.%, gewählt wird.
Ethylhexylglycerin ist vorteilhaft zugesetzt zu einem Anteil von 0,001 - 1 Gew.%, bevorzugt 0,01 - 0,2 Gew.%, besonders bevorzugt 0,05 - 0,1 Gew.%, jeweils bezogen auf die Gesamtmasse der Zubereitung.
Auch die Verwendung einer Makroemulsion, insbesondere mit einer Tropfengröße im Bereich von 0,1 bis 10 µm und insbesondere mit flüssigkristalliner Struktur, umfassend ein oder mehrere amphiphile Wirkstoffe, insbesondere desodorierende Wirkstoffe, wobei als amphiphiler Wirkstoff Octenidindihydrochlorid im Bereich von 0,0001 bis 0,03 Gew.%, besonders bevorzugt im Bereich von 0,005 bis 0,015 Gew.%, jeweils bezogen auf die Gesamtmasse der Zubereitung, zeigt eine Verbesserung der Wirksamkeit dieser Wirkstoffe auf oder in der Haut.

Ebenso ist die Verwendung von Makro-Emulsionen und einem oder mehreren amphiphilen Wirkstoffen zur Herstellung kosmetischer oder dermatologischer Zubereitungen mit verbesserter Wirksamkeit beschrieben. Eine antimikrobielle und damit Deowirksamkeit ist für amphiphile Wirkstoffe, wie Octenidine, nur aus Makroemulsion gegeben. Bei der Mikroemulsion ist die *in vivo*-Wirkleistung für den Verbraucher nicht ausreichend/ zufriedenstellend. Damit ist erst die Kombination aus amphiphilen Wirkstoffen und Makroemulsion, insbesondere mit einer Tropfengröße im Bereich von 0,1 bis 10 µm und

insbesondere mit flüssigkristalliner Struktur, geeignet, eine verbesserte, d.h. ausreichende Deowirksamkeit, zu erreichen.

In einer besonderen Ausführungsform umfassen die erfindungsgemäßen Zubereitungen Deodorantformulierungen.

Die Zubereitung kann zusätzlich zu den amphiphilen Wirkstoffen weitere Wirkstoffe, unabhängig von ihrer Polarität umfassen.

Den erfindungsgemäßen Zubereitungen können vorteilhaft zur weiteren Unterstützung der desodorierenden Wirkleistung antimikrobielle Wirkstoffe zugesetzt werden. Hier sind bevorzugt naturidentische Rohstoffe, wie Butyloctansäure oder Propylenglycol, zu wählen. Bevorzugt sind den erfindungsgemäßen Zubereitungen antitranspirant-wirksame Stoffe wie vicinale Diole (wie in DE 10200904269 A1 beschrieben), bevorzugt 1,2-Propylenglykol, und/oder Salze von 1,1-Dimethyl-piperidinium oder 1,1-Dimethyl-pyrrolindium Verbindungen wie bspw. 4-[(2-Cyclopentyl-2-hydroxyphenylacetyl)oxy]-1,1-dimethyl-piperidiniumbromid oder andere Salze, 4-[(2-Cyclohexyl-2-hydroxyphenylacetyl)oxy]-1,1-dimethyl-piperidiniumbromid oder andere Salze, 4-[(3-Methyl-2-hydroxyphenylpentanoyl)oxy]-1,1-dimethyl-piperidiniumbromid oder andere Salze, 4-[(3-Methyl-2-phenylpentanoyl)oxy]-1,1-dimethyl-piperidiniumbromid oder andere Salze oder auch 3-[(2-Cyclopentyl-2-hydroxyphenylacetyl)oxy]-1,1-dimethylpyrrolidiniumbromid zugesetzt.
Bevorzugt umfassen die erfindungsgemäßen Zubereitungen neben Octenidine Propylenglycol und Ethylhexylglycerin, vorteilhaft in den angegeben Anteilsbereichen.
Der Anteil an Butyloctansäure beträgt vorteilhaft 0,01 bis 2 Gew.%, bevorzugt 0,01 - 0,5 Gew.%, besonders bevorzugt 0,1 Gew.%, jeweils bezogen auf die Gesamtmasse der Zubereitung.
Der Anteil an vicinale Diolen, speziell 1,2-Propylenglykol, beträgt vorteilhaft 10 bis 50 Gew.%, bevorzugt 15 - 30 Gew.%, besonders bevorzugt 20 Gew.%, jeweils bezogen auf die Gesamtmasse der Zubereitung.
Die erfindungsgemäße Makroemulsion kann der Fachmann aus den ihm bekannten Bestandteilen herstellen. Als bevorzugte Emulgatoren zur Herstellung der erfindungsgemäßen Makroemulsionen werden Steareth-2, Steareth-21 und Polyglyceryl-3 Methylglucose Distearat gewählt, bevorzugt Steareth-2 und Steareth-21 zusammen als Emulgatorsystem. Wird erfindungsgemäß bevorzugt eine Wasser-in-Öl Makroemulsion in Aerosolform gewählt, sind Cetyl PEG/PPG-10/1 Dimethicone, Sorbitan Trioleate als Emulgatoren bevorzugt.
Vorteilhaft werden diese Emulgatoren zu einem Anteil von 0,5 bis 5 Gew.%, bezogen auf die Gesamtmasse der Zubereitung, eingesetzt. Vorteil dabei ist, dass in dieser Anteilsmenge die in der Zubereitung vorhandenen Lipide nicht einfach zu kleinen Tröpfchen emulgiert werden können und damit die Bildung einer erfindungsgemäßen Makroemulsion unterstützt wird.

Die Zubereitung umfasst bevorzugt keine antitranspirant-wirksamen Aluminiumsalze, wie Aluminiumchlorohydrate.

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, sofern der Zusatz die geforderten Eigenschaften hinsichtlich der desodorierenden Wirkung nicht beeinträchtigt.

Bevorzugt umfassen die erfindungsgemäßen Zubereitungen ein oder mehrere weitere antimikrobiell wirksame Mittel.

Ebenso bevorzugt wird auf den Zusatz an Konservierungsmittel, Konservierungshelfer verzichtet. Die Formulierungen sind dann Konsumentenwünschen entsprechend konservierungsmittelfrei.
Bekannte Konservierungsmittel sind die als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben.
Des Weiteren ist Phenoxyethanol, Ethylenglycolmonophenylether, Phenylglycol, Phenoxetol^{®} zur Konservierung von kosmetischen Mitteln bekannt.
Auf diese Stoffe kann erfindungsgemäß bevorzugt verzichtet werden.

Erfindungsgemäß bevorzugte kosmetische oder dermatologische Zubereitungen sind in Form von Roll-on, Zerstäuber und Aerosolen als Makroemulsion zu formulieren, umfassend ein oder mehrere amphiphile Deodorantwirkstoffe, insbesondere Octenidine.
Bevorzugt sind bei den Aerosolen W/O-Makroemulsionsformen.

Im erfindungsgemäß bevorzugten Aerosol wird als Treibgas bevorzugt Propan, Propen, n-Butan, iso-Butan, iso-Buten, n-Pentan, n-Penten, iso-Pentan, iso-Penten, Methan, Ethan, Dimethylether, Stickstoff, Luft, Sauerstoff, Stickstoffoxide, Lachgas, 1,1,1,3-Tetrafluorethan, Heptafluoro-n-propan, Perfluorethan, Monochlordifluormethan, 1,1-Difluorethan einzeln oder in Kombination eingesetzt. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt.
Besonders bevorzugt sind Propan, Butan, iso-Butan bzw. Mischungen dieser Treibgase. Die genannten Gase können im Sinne der vorliegenden Erfindung jeweils einzeln oder in beliebigen Mischungen zueinander verwendet werden. Bevorzugt werden Mischungen mit Druckstufen von 2,0 bis 3,5 bar eingesetzt. Ganz besonders bevorzugt ist eine Druckstufe von 2,7 bar.

Die folgenden Beispiele sollen die Erfindung näher beschreiben, ohne einschränkend zu wirken.

Die Anteilsangaben beziehen sich auf Gewichtsanteile, sofern nichts anderes offenbart ist.

### Beispiele

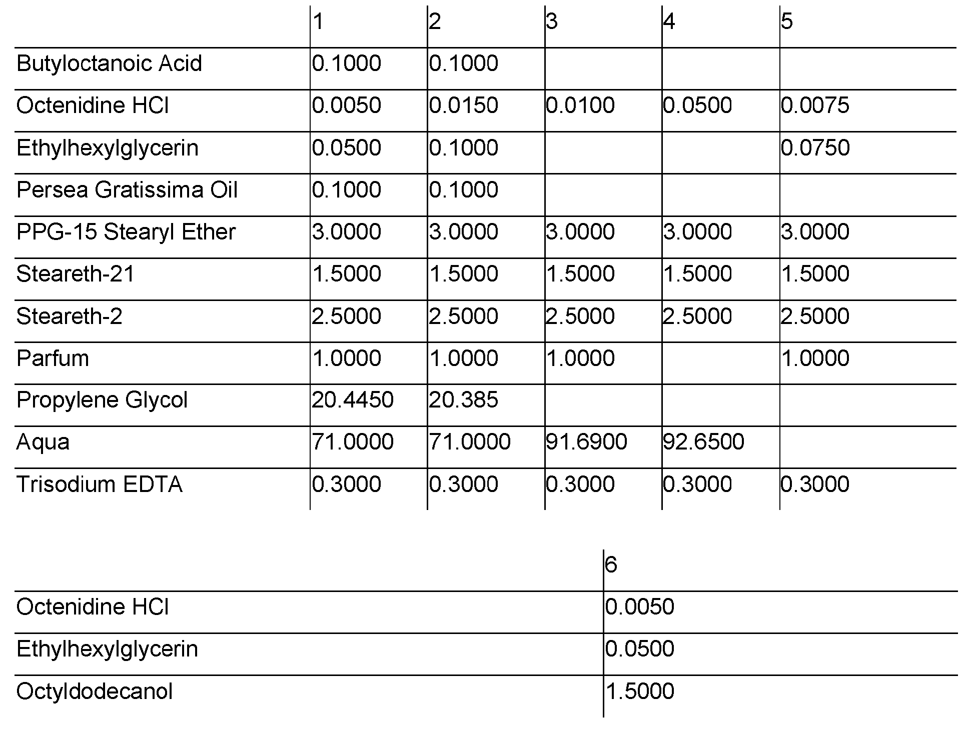

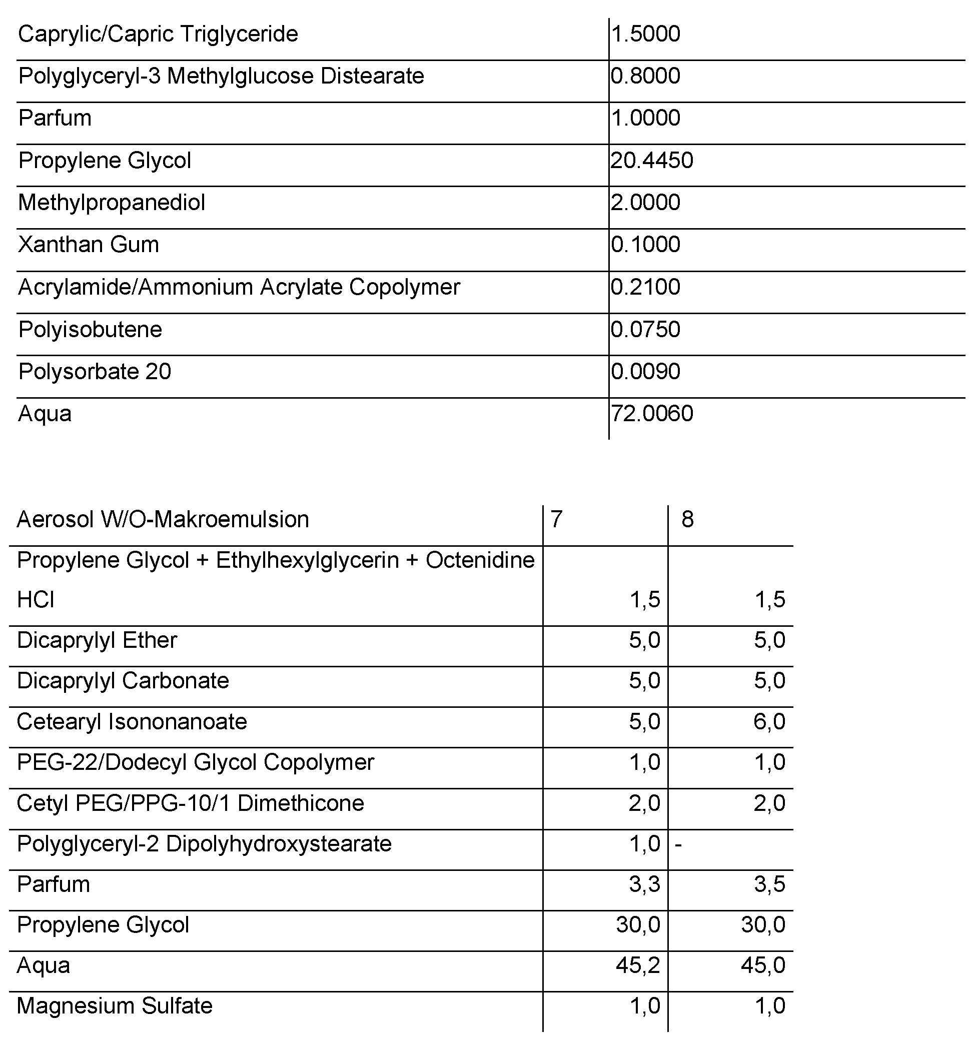

Abfüllverhältnis 30:70 (Treibgasmischung aus Butan, Propan, Isobutan, Druckstufe 2.7)

## Patentansprüche

1. Kosmetische oder dermatologische Zubereitungen auf Basis einer Makro-Emulsion umfassend ein oder mehrere amphiphile, insbesondere desodorierende Wirkstoffe, **dadurch gekennzeichnet, dass** als amphiphiler Wirkstoff Octenidindihydrochlorid gewählt wird und der Anteil an Octenidindihydrochlorid im Bereich von 0,0001 - 0,03 Gew.%, jeweils bezogen auf die Gesamtmasse der Zubereitung, gewählt wird.

2. Zubereitung nach Anspruch 1 **dadurch gekennzeichnet, dass** der Anteil an Octenidinhydrochlorid im Bereich von 0,005 - 0,015 Gew.%, jeweils bezogen auf die Gesamtmasse der Zubereitung, gewählt wird.

3. Zubereitung nach einem der vorstehenden Ansprüche umfassend ein oder mehrere weitere antimikrobiell wirksame Mittel.

4. Zubereitung nach einem der vorstehenden Ansprüche umfassend Butyloctansäure, Propylenglycol und/oder Ethylhexylglycerin.

5. Zubereitung nach einem der vorstehenden Ansprüche umfassend keine antitranspirant-wirksamen Aluminiumsalze.

6. Zubereitung nach einem der vorstehenden Ansprüche umfassend keine Konservierungsmittel und/oder Konservierungshelfer, insbesondere keine als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben, sowie kein Phenoxyethanol, Ethylenglycolmonophenylether und/oder Phenylglycol.

7. Zubereitung nach einem der vorstehenden Ansprüche umfassend ein oder mehrere Emulgatoren, gewählt aus der Gruppe Steareth-2, Steareth-21, Polyglyceryl-3 Methylglucose Distearat, Cetyl PEG/PPG-10/1 Dimethicone, Sorbitan Trioleate.

8. Zubereitung nach Anspruch 7, **dadurch gekennzeichnet, dass** der Anteil der Emulgatoren im Bereich von 0,5 bis 5 Gew.%, bezogen auf die Gesamtmasse der Zubereitung, gewählt wird.

9. Zubereitung nach einem der vorstehenden Ansprüche als Wasser-in-Öl Emulsion.

10. Zubereitung nach einem der vorstehenden Ansprüche in Form eines Aerosols, Roll-ons, oder Zerstäubers, insbesondere Aerosol.

## Claims

1. Cosmetic or dermatological preparations based on a macroemulsion comprising one or more amphiphilic, in particular deodorizing active ingredients, **characterized in that** the amphiphilic active ingredient selected is octenidine dihydrochloride and the fraction of octenidine dihydrochloride is selected in the range from 0.0001-0.03% by weight, in each case based on the total mass of the preparation.

2. Preparation according to Claim 1, **characterized in that** the fraction of octenidine hydrochloride is selected in the range from 0.005-0.015% by weight, in each case based on the total mass of the preparation.

3. Preparation according to one of the preceding claims, comprising one or more further antimicrobially effective agents.

4. Preparation according to one of the preceding claims, comprising butyloctanoic acid, propylene glycol and/or ethylhexylglycerin.

5. Preparation according to one of the preceding claims, comprising no antiperspirant-effective aluminium salts.

6. Preparation according to one of the preceding claims, comprising no preservatives and/or preservation aids, in particular no compounds referred to as parabens, in particular 4-hydroxybenzoic acid and esters thereof, methylparaben, ethylparaben, propylparaben, isopropylparaben, butylparaben, isobutylparaben, phenylparaben, as well as no phenoxyethanol, ethylene glycol monophenyl ether and/or phenyl glycol.

7. Preparation according to one of the preceding claims, comprising one or more emulsifiers selected from the group steareth-2, steareth-21, polyglyceryl-3 methylglucose distearate, cetyl PEG/PPG-10/1 dimethicone, sorbitan trioleate.

8. Preparation according to Claim 7, **characterized in that** the fraction of the emulsifiers is selected in the range from 0.5 to 5% by weight, based on the total mass of the preparation.

9. Preparation according to one of the preceding claims as water-in-oil emulsion.

10. Preparation according to one of the preceding claims in the form of an aerosol, roll-on, or atomizer, in particular aerosol.

## Revendications

1. Préparations cosmétiques ou dermatologiques à base d'une macroémulsion comprenant une ou plusieurs substances actives amphiphiles, en particulier désodorisantes, **caractérisées en ce qu'**on choisit, comme substance active amphiphile, du dichlorhydrate d'octénidine et la proportion de dichlorhydrate d'octénidine est choisie dans la plage de 0,0001-0,03% en poids, à chaque fois par rapport à la masse totale de la préparation.

2. Préparation selon la revendication 1, **caractérisée en ce que** la proportion de chlorhydrate d'octénidine est choisie dans la plage de 0,005-0,015% en poids, à chaque fois par rapport à la masse totale de la préparation.

3. Préparation selon l'une quelconque des revendications précédentes, comprenant un ou plusieurs agents actifs de manière antimicrobienne.

4. Préparation selon l'une quelconque des revendications précédentes, comprenant de l'acide butyloctanoïque, du propylèneglycol et/ou de l'éthylhexylglycérol.

5. Préparation selon l'une quelconque des revendications précédentes, ne comprenant pas de sels d'aluminium actifs de manière antitranspirante.

6. Préparation selon l'une quelconque des revendications précédentes, ne comprenant pas de conservateurs et/ou d'adjuvants de conservation, en particulier pas de composés appelés parabènes, en particulier l'acide 4-hydroxybenzoïque et ses esters, le méthylparabène, l'éthylparabène, le propylparabène, l'isopropylparabène, le butylparabène, l'isobutylparabène, le phénylparabène, et ne contenant pas de phénoxyéthanol, d'éthylèneglycolmonophényléther et/ou de phénylglycol.

7. Préparation selon l'une quelconque des revendications précédentes, comprenant un ou plusieurs émulsifiants, choisis dans le groupe formé par le stéareth-2, le stéareth-21, le polyglycéryl-3 méthylglucose distéarate, le cétyl-PEG/PPG-10/1 diméthicone, le trioléate de sorbitane.

8. Composition selon la revendication 7, **caractérisée en ce que** la proportion d'émulsifiants est choisie dans la plage de 0,5 à 5% en poids, par rapport à la masse totale de la préparation.

9. Préparation selon l'une quelconque des revendications précédentes sous forme d'émulsion huile-dans-eau.

10. Préparation selon l'une quelconque des revendications précédentes sous forme d'un aérosol, d'un applicateur à bille ("roll-on") ou d'un pulvérisateur, en particulier sous forme d'un aérosol.
